# EUROPEAN PATENT APPLICATION

(11) **EP 4 083 210 A1**
(43) Date of publication of application: **02.11.2022**
(21) Application number: 20907917.7
(22) Date of filing: 07.12.2020
(51) Int. Cl.: C12N 15/115, A61K 48/00, A61K 31/713, A61P 35/00

(54) **ANTI-CANCER IMMUNOTHERAPEUTIC COMPOSITION FOR TREATING CANCER**

(30) Priority: 27.12.2019 KR 20190176287
(71) Applicant: Interoligo Corporation, Gyeonggi-do 14058 (KR)
(72) Inventor: LEE, Jung Hwan, Yongin-si, Gyeonggi-do 17103 (KR); LEE, Jong Ook, Seongnam-si, Gyeonggi-do 13457 (KR); KIM, Jin Woo, Daejeon 34006 (KR)
(74) Representative: dompatent von Kreisler Selting Werner - Partnerschaft von Patent- und Rechtsanwälten mbB
(86) International application number: PCT/KR2020/017757
(87) International publication number: WO 2021/132936

(57) **Abstract**

The present invention relates to an aptamer or a functional fragment thereof, capable of specifically binding to PD-L1 or capable of inhibiting interaction between PD-1 and PD-L1 by specifically binding to PD-L1, which includes a nucleic acid sequence of SEQ ID NO. 1, 2, 10 or 18.

## Description

### [Technical Field]

The present invention relates to an immunotherapeutic composition including an aptamer.

### [Background Art]

Cancer is known as one of the major causes of death worldwide. Although surgical excision and conventional chemotherapy are commonly used, surgery has a high recurrent rate and side effects, while chemotherapy involves clinical problems to be solved. Therefore, it is necessary to develop new methods and means for treating cancer patients so as to increase a survival time of the patients.

Immunochemotherapy is a new anticancer treatment method that has recently received a great attention, and is a therapy that activates the immune system of a human body so that immune cells attack cancer cells to acquire anticancer effects. Currently, immune anticancer therapeutics ("immunochemo-therapeutics") approved to be available on the market are mostly immune checkpoint inhibitors. T cells as one of immune cells of the human body are activated by the action of tumor-specific antigens expressed on the surface of cancer cells, and have a role of recognizing, attacking and killing cancer cells. The cancer cells use various immune checkpoint proteins as a mechanism to evade such attack of T cells. For example, programmed death-ligand 1 (PD-L1) protein expressed in cancer cells binds to programmed cell death protein 1 (PD-1) expressed in T cells and transmits an inhibitory signal to inactivate T cells, thereby avoiding the attack of T cells. The immune checkpoint inhibitors activate T cells by inhibiting an immune evasion mechanism of cancer cells using a method of blocking the binding between various types of immune checkpoint proteins, thereby removing cancer cells. Targets of the currently used immune checkpoint inhibitors include CTLA-4, PD-1, PD-L1, or the like, and antibody-based anticancer drugs that inhibit signal transduction of the above inhibitors, such as Yervoy, Optivo, Thycentric, etc. belong to the above category (Ott et al. (2013) Clin Cancer Res. 19:5300-5309).

A biomarker refers to a protein, DNA, RNA or metabolite that is objectively useable to distinguish pathological status or progression of diseases including cancer or to predict a therapeutic response. In particular, the development or diagnosis of biomarkers capable of distinguishing responders and non-responders to a specific drug and monitoring therapeutic effects has become a very important factor in dividing the success or failure of new anticancer drugs which are now being developed. This is because predicting patient treatment effects has emerged as a very important factor, not only in the scientific aspect that new anticancer drugs use a method of attacking specific molecular and biological changes in cancer but also in the economical aspect due to the drug price being too high compared to the existing chemotherapy.

In the case of some immune checkpoint inhibitors, it is necessary to perform a companion diagnosis that examines the expression of target proteins in cancer tissues by immunological methods using living tissues of a cancer patient. However, a problem that a correlation between the expression of the above protein and therapeutic effects thereof is not clear has already been pointed out. This may be due to different reasons such as the sensitivity of the immunological method used for companion diagnosis and the effectiveness of biomarkers. Therefore, a number of researches have done on development of various high-sensitivity diagnostic methods, however, no obvious results have been found yet (Havel et al. (2019) Nat Rev Cancer 19: 133-150).

An aptamer is a molecule having a single-stranded oligonucleotide sequence, may have a specific three-dimensional structure through a hydrogen bonding in the molecule, and thus may specifically bind to a target through the three-dimensional spatial structure. Since the aptamer is target-specific, some aptamers can inhibit functions of target substances. Further, the aptamer binds to a target thus to block an intracellular signal transduction pathway. Accordingly, the aptamer has attracted attention as a nucleic acid medicine for specifically inhibiting a target.

Aptamers may be selected through systematic evolution of ligands by exponential enrichment (SELEX). The aptamer may bind to a target by forming a specific three-dimensional structure, and have high affinity, high specificity and ease of synthesis, as well as an advantage of no or little immunogenicity. Since some aptamers can not only bind to a target substance but also inhibit functions of the target substance, various applications are possible in terms of disease treatment. Till now, the FDA has approved pegaptanib sodium, which is a nucleic acid aptamer specifically binding to VEGF, as a therapeutic agent for age-related macular degeneration. Further, in the field of tumor treatment, aptamers targeting VEGF, PDGF, nucleolin, etc. have entered clinical trial for the purpose of cancer treatment (Dunn et al. (2017) Nat Rev Chem 1:76, Ng et al. (2006) Nat Rev Drug Discovery 5: 123-132).

### [Summary of Invention]

### [Problems to be Solved by Invention]

An object of the present invention is to provide an aptamer for treatment of cancer with immunotherapy.

Another object of the present invention is to provide an aptamer capable of detecting cancer cells.

### [Means for Solving Problems]

1. An aptamer or a functional fragment thereof, capable of specifically binding to programmed death-ligand 1 (PD-L1) or capable of inhibiting interaction between programmed cell death protein 1 (PD-1) and PD-L1 by specifically binding to PD-L1, the aptamer including a nucleic acid sequence of SEQ ID NO: 1, 2, 10 or 18.

2. The aptamer or a functional fragment thereof according to item 1, wherein the functional fragment is any one of SEQ ID NOs: 3 to 9, and 11 to 17.

3. A pharmaceutical composition for treating cancer, including one or more of an aptamer or a functional fragment thereof, the aptamer including a nucleic acid sequence selected from the group consisting of SEQ ID NOs: 1, 2, 10, and 18.

4. The pharmaceutical composition according to item 3, wherein the functional fragment is any one of SEQ ID NOs: 3 to 9, and 11 to 17.

5. The pharmaceutical composition according to item 3, wherein the functional fragment is SEQ ID NO: 3, 4, 5, 6, 7, 9, 11, 12, 14 or 16.

6. The pharmaceutical composition according to item 3, wherein the cancer is one in which PD-L1 is over-expressed.

7. The pharmaceutical composition according to item 3, wherein the cancer is prostate cancer, gallbladder cancer, intrahepatic biliary tract cancer, biliary tract cancer, oral cancer, pharyngeal cancer, laryngeal cancer, tongue cancer, duodenal cancer, eye tumor, mediastinal cancer, sinus cancer, renal pelvic cancer, heart cancer, glioblastoma, neuroblastoma, liver cancer, bone cancer, pancreatic cancer, skin cancer, head and neck cancer, breast cancer, lung cancer, skin or intraocular malignant melanoma, kidney cancer, uterine cancer, ovarian cancer, colon cancer, rectal cancer, anal region cancer, colorectal cancer, stomach cancer, testicular cancer, fallopian tube carcinoma, endometrial carcinoma, cervical carcinoma, vaginal carcinoma, vulvar cancer, non-Hodgkin's lymphoma, esophageal cancer, small intestinal cancer, endocrine system cancer, thyroid cancer, parathyroid cancer, adrenal gland cancer, soft tissue sarcoma, urethral cancer, penile cancer, childhood cancer, lymphocytic lymphoma, bladder cancer, ureter cancer, renal pelvic carcinoma, central nervous system (CNS) cancer, primary CNS lymphoma, tumor angiogenesis, spinal cancer, brainstem glioma, pituitary adenoma, Kaposi's sarcoma, epidermal cancer, squamous cell carcinoma, multiple myeloma, B-cell lymphoma, Hodgkin's lymphoma, acute myeloid lymphoma, chronic myeloid leukemia, chronic lymphocytic leukemia, follicular lymphoma, diffuse large B-cell lymphoma, Burkitt's lymphoma, immune large-cell lymphoma, progenitor B-lymphoblastic lymphoma, mantle cell lymphoma, acute lymphoblastic lymphoma, mycosis fungoides, anaplastic large-cell lymphoma, acute myeloid leukemia, acute lymphoblastic leukemia, hepatoblastoma, retinoblastoma, peritoneal cancer, brain tumor, thymic cancer, T-cell lymphoma or precursor T-lymphoblastic lymphoma.

8. The pharmaceutical composition according to item 3, wherein at least one of a small molecule, a nucleic acid, a protein and a radioisotope is bound to the 5'-terminus, 3'-terminus or both thereof in the aptamer or the functional fragment thereof.

9. A composition for detecting cancer cells, including at least one of an aptamer or a functional fragment thereof, the aptamer including a nucleic acid sequence selected from the group consisting of SEQ ID NOs: 1, 2, 10 and 18.

10. The composition for detection according to item 9, wherein the functional fragment is any one of SEQ ID NOs: 3 to 9, and 11 to 17.

11. The composition according to item 9, wherein the functional fragment is SEQ ID NO: 3, 4, 5, 6, 7, 9, 11, 12, 14 or 16.

12. The composition according to item 9, wherein the cancer cells are cells in which PD-L1 is over-expressed.

13. The composition according to item 9, wherein the cancer is prostate cancer, gallbladder cancer, intrahepatic biliary tract cancer, biliary tract cancer, oral cancer, pharyngeal cancer, laryngeal cancer, tongue cancer, duodenal cancer, eye tumor, mediastinal cancer, sinus cancer, renal pelvic cancer, heart cancer, glioblastoma, neuroblastoma, liver cancer, bone cancer, pancreatic cancer, skin cancer, head and neck cancer, breast cancer, lung cancer, skin or intraocular malignant melanoma, kidney cancer, uterine cancer, ovarian cancer, colon cancer, rectal cancer, anal region cancer, colorectal cancer, stomach cancer, testicular cancer, fallopian tube carcinoma, endometrial carcinoma, cervical carcinoma, vaginal carcinoma, vulvar cancer, non-Hodgkin's lymphoma, esophageal cancer, small intestinal cancer, endocrine system cancer, thyroid cancer, parathyroid cancer, adrenal gland cancer, soft tissue sarcoma, urethral cancer, penile cancer, childhood cancer, lymphocytic lymphoma, bladder cancer, ureter cancer, renal pelvic carcinoma, central nervous system (CNS) cancer, primary CNS lymphoma, tumor angiogenesis, spinal cancer, brainstem glioma, pituitary adenoma, Kaposi's sarcoma, epidermal cancer, squamous cell carcinoma, multiple myeloma, B-cell lymphoma, Hodgkin's lymphoma, acute myeloid lymphoma, chronic myeloid leukemia, chronic lymphocytic leukemia, follicular lymphoma, diffuse large B-cell lymphoma, Burkitt's lymphoma, immune large-cell lymphoma, progenitor B-lymphoblastic lymphoma, mantle cell lymphoma, acute lymphoblastic lymphoma, mycosis fungoides, anaplastic large-cell lymphoma, acute myeloid leukemia, acute lymphoblastic leukemia, hepatoblastoma, retinoblastoma, peritoneal cancer, brain tumor, thymic cancer, T-cell lymphoma or precursor T-lymphoblastic lymphoma.

14. The composition according to item 9, wherein at least one of a fluorescent label, a Raman scattering label, an enzyme label, an infrared label and a radiolabel is bound to the 5'-terminus, 3'-terminus or both thereof in the aptamer or the functional fragment thereof.

### [Advantageous effects]

The composition including the aptamer according to the present invention can treat cancer, alleviate symptoms of cancer, or prevent cancer.

The composition including the aptamer according to the present invention can detect cancer cells, in particular, cancer cells in which PD-L1 is over-expressed.

### [Brief Description of Drawings]

FIG. 1 illustrates results of measuring the binding force of an aptamer to a target protein according to an embodiment of the present invention.
FIG. 2 illustrates results of confirming PD-1/PD-L1 binding inhibitory activity of the aptamer and PD-1/PD-L1 binding inhibitory activity of the anti-PD-Ll antibody according to an embodiment of the present invention.
FIG. 3 illustrates results of confirming PD-1/PD-L1 binding inhibitory activity of the aptamer according to an embodiment of the present invention.
FIG. 4 illustrates results of confirming PD-1/PD-L1 binding inhibitory activity of an aptamer according to another embodiment of the present invention.
FIG. 5 illustrates results of confirming PD-1/PD-L1 binding inhibitory activity of the aptamer and PD-1/PD-L1 binding inhibitory activity of the anti-PD-Ll antibody according to an embodiment of the present invention.
FIG. 6 illustrates PD-1/PD-L1 binding inhibitory activity of the aptamer and PD-1/PD-L1 binding inhibitory activity of the anti-PD-Ll antibody according to another embodiment of the present invention.

### [Mode for Carrying out Invention]

Hereinafter, the present invention will be more fully described with reference to the accompanying drawings, wherein only some, but not all, embodiments of the present invention are illustrated. Indeed, the present invention may be embodied in many different forms and should not be construed as being limited to the embodiments set forth herein. As used in this specification and the appended claims, the singular expressions also include plural expressions unless clearly indicated otherwise.

An aptamer is a single-stranded oligonucleotide (ssDNA, RNA, modified DNA, or modified RNA). Such an aptamer has a specific three-dimensional structure, and exhibits high binding force and high specificity to a target substance. Although these characteristics are functions similar to those of an antibody composed of a protein, it has been reported that the aptamer exhibits low immunogenicity compared to the antibody when administered to the body. Further, unlike biologically produced antibodies, chemically synthesized aptamers are easy to modify *in vitro* and minimize a difference between production batches, which in turn is advantageous for drug development and mass production compared to antibodies. On the other hand, since aptamers have higher stability than antibodies, they have an advantage of easy preservation during manufacturing products.

Since such an aptamer specifically binds to a target, some aptamers may inhibit functions of target substances or block the signal transduction of cells by binding to the target substances. In particular, in the treatment of tumors, aptamers targeting VEGF, PDGF or nucleolin are included in the clinical research pipeline, which reflects the expectations of nucleic acid aptamers in tumor therapy.

In particular, the aptamer according to the present invention may include a nucleic acid sequence of SEQ ID NO: 1, 2, 10 or 18 that can specifically bind to programmed death-ligand 1 (PD-L1) or can specifically bind to PD-L1 and inhibit the interaction between programmed cell death protein 1 (PD-1) and PD-L1, and may be a functional fragment of SEQ ID NO: 1, 2, 10 or 18.

In the present invention, the functional fragment refers to a fragment in which the binding ability of an aptamer parent to the target substance is enhanced or maintained, or in which the 5'-terminus and/or 3'-terminus substantially having the binding ability to the target substance is partially deleted. For example, when there is an aptamer parent that binds to PD-L1 and has a length of 74mer, 73mer to 20mer-length aptamers with deletion of 5'- terminus and/or 3'-terminus and having the binding ability to PD-L1 are referred to as functional fragments.

Aptamers can be selected by a conventional screening method, that is, systematic evolution of ligands by exponential enrichment (SELEX). Although the SELEX method is a screening method of nucleic acid aptamers, the screening of useful aptamers in practice still remains difficult in the art, and commercially available aptamer-based drugs effective for treating specific cancers have yet to be approved for marketing.

A typical SELEX method for screening aptamers will be described below. However, those skilled in the art may add, modify or delete each step and use the same on the basis of knowledge and skill known in the art.

A single-stranded DNA library is prepared such that 40 to 60 consecutive random sequences are usually in a central portion of the single-stranded DNA and biotin may be bound to the 5'-terminus. Negative selection is prepared by binding the same to a non-target. More specifically, the single-stranded DNA library may be mixed with the non-target by putting the single-stranded DNA library into a reaction tank in which the non-target is contained. At this time, the non-target may be, for example, a protein abundant in plasma, but it is not limited thereto. The protein having a structure similar to the target protein expressed in non-tumor tissues may be used. During the binding reaction between the single-stranded library and the non-target, single-stranded DNA sequences of the single-stranded DNA library specific to the non-target are bound. In this embodiment, the binding reaction is performed for, for example, 1 minute to 60 minutes, or about 30 minutes.

Thereafter, the non-target is immobilized on magnetic beads to isolate single-stranded DNA sequences that do not bind to the non-target. More specifically, the magnetic beads capable of binding histidine tag prepared by washing are reacted with a mixture of a non-target protein and a single-stranded DNA library. The binding reaction is performed for, for example, 1 minute to 60 minutes, or about 15 minutes. Single-stranded DNA sequences not bound to non-target proteins may be isolated by separating the supernatant since they remain unbound to the magnetic beads.

Positive selection is performed by binding a single-stranded DNA that does not bind to the non-target ("not binding to the non-target") to a target. More specifically, a single-stranded DNA sequence not binding to the non-target protein is placed in the reaction tank containing a target protein, followed by mixing the same. During the reaction of the single-stranded DNA sequence with the target protein, the single-stranded DNA sequence having specificity to the target protein binds to the target protein. In this embodiment, the binding reaction is performed for, for example, 1 minute to 60 minutes, or about 30 minutes.

Thereafter, the target immobilized on the magnetic beads is washed to remove single-stranded DNA sequences that have low binding affinity to the target or do not bind to the target. More specifically, magnetic beads are added to the mixture that induced binding of the target protein to the single-stranded DNA sequence, followed by immobilizing the target protein to the magnetic beads using a tag of the target protein. A washing buffer is injected into the reaction tank to wash the magnetic beads immobilized with the target protein. During the washing step, single-stranded DNA sequences not binding to the target protein may be removed because the single-stranded DNA sequence binding to the target protein still remains bound to the magnetic beads. In one embodiment, the washing step may be performed once or multiple times as needed.

The single-stranded DNA sequence is separated by heating the target to which the single-stranded DNA sequence is bound or by treatment with a sodium hydroxide solution. In one embodiment, the heating temperature may be, for example, 92 °C, 95 °C or 98 °C, but it is not limited thereto. A concentration in sodium hydroxide treatment may be selected from a concentration between 1 mM and 30 mM. Since the isolated single-stranded DNA sequence is specific to the target protein, it may be referred to as an aptamer specific to the target protein.

The isolated single-stranded DNA sequence is subjected to amplification. In one embodiment, the method for amplifying the isolated single-stranded DNA may be, for example, PCR, which can be performed using the isolated single-stranded DNA sequence as a template and using a 5'-primer or a 3'-primer.

In one embodiment, the PCR product obtained through the above process may be selected multiple times. More specifically, the obtained PCR product is used as a single-stranded DNA library for the next step of selection, and the above steps are repeated to select a single-stranded DNA sequence with higher specificity to the target protein. After repeating the selection process several times, the obtained PCR product is subjected to cloning, followed by DNA sequence analysis so as to acquire an aptamer having high specificity to the target protein.

A SELEX method according to another embodiment of the present invention will be described below. However, those skilled in the art may add, modify or delete each step of the method and then use the same on the basis of knowledge and skill known in the art.

A single-stranded DNA library is prepared such that 40 to 60 consecutive random sequences may be positioned in the center of the single-stranded DNA. Negative selection is performed by binding the library to non-target diseased tissue sections. More specifically, the single-stranded DNA library is mixed with the non-target diseased tissue sections by placing the single-stranded DNA library in a reaction tank in which the non-target diseased tissue sections are contained. In this case, the non-target diseased tissue section may be, for example, a normal tissue section, but it is not limited thereto. Non-tumor tissue sections are also able to be used as non-target diseased tissue sections. During the binding reaction between the single-stranded library and the non-target diseased tissue sections, single-stranded DNA sequences of the single-stranded DNA library specific to the non-target diseased tissue sections may bind to the non-target diseased tissue sections. In this embodiment, the binding reaction is performed for, for example, 1 minute to 60 minutes, or about 30 minutes.

Following this, the non-target diseased tissue sections are washed to isolate single-stranded DNA sequences not binding to the non-target diseased tissue sections. More specifically, a wash buffer is injected into the reaction tank to wash the non-target diseased tissue sections. During the washing step, the single-stranded DNA sequences not binding to the non-target diseased tissue sections can be isolated because the single-stranded DNA sequences binding to the non-target diseased tissue sections still remain bound to the non-target diseased tissue sections.

Positive selection is performed by combining single-stranded DNA not binding to a non-target diseased tissue section with the target diseased tissues. More specifically, the single-stranded DNA sequence not binding to the non-target diseased tissue section is introduced to a reaction tank containing the target diseased tissue sections, followed by mixing the single-stranded DNA sequence not binding to the non-target diseased tissue section with the target diseased tissue section. During a reaction of the single-stranded DNA sequence with the target diseased tissue section, the single-stranded DNA sequence specific to the target diseased tissue section may bind to the target diseased tissue section. In this embodiment, the binding reaction is performed for, for example, 1 minute to 60 minutes, or about 30 minutes.

Thereafter, the target diseased tissue section is washed to remove single-stranded DNA sequences that have low binding affinity to the target diseased tissue section or do not bind to the target diseased tissue section. More specifically, a wash buffer is injected into the reaction tank to wash the target diseased tissue sections. During the washing step, single-stranded DNA sequences not binding to the target diseased tissue sections can be removed because the single-stranded DNA sequence binding to the target diseased tissue section still remains bound to the target diseased tissue section. In one embodiment, the washing step may be performed once or multiple times as needed.

By heating the target diseased tissue section to which the single-stranded DNA sequence is bound, the single-stranded DNA sequence is isolated. In one embodiment, the heating temperature may be, for example, 92 °C, 95 °C or 98 °C, but it is not limited thereto. Since the isolated single-stranded DNA sequence is specific to the target diseased tissue section, it may be referred to as an aptamer specific to the target disease.

The isolated single-stranded DNA sequence is subjected to amplification. In one embodiment, a method for amplifying the isolated single-stranded DNA may be, for example, PCR, which can be performed using the isolated single-stranded DNA sequence as a template and using a 5'-primer or a 3'-primer.

In one embodiment, the PCR product obtained through the above process may be selected multiple times. More specifically, the obtained PCR product is used as a single-stranded DNA library for the next step of selection, and the above steps are repeated to select a single-stranded DNA sequence with higher specificity to the target protein. After repeating the selection process several times, the obtained PCR product is subjected to cloning, followed by DNA sequence analysis so as to acquire an aptamer having high specificity to the target protein.

As used herein, the terms "nucleic acid", "nucleic acid molecule", "oligonucleotide" and "polynucleotide" are used interchangeably, and refer to a polymeric form of phosphoric ester or any phosphoric ester analogs or derivatives thereof such as phosphorothioate and thioester of: ribonucleosides (adenosine, guanosine, uridine or cytidine; "RNA molecule"); or deoxyribunucleosides (deoxyadenosine, deoxyguanosine, deoxythymine, deoxycytidine or a nucleic acid derivative; "DNA molecule") in single-stranded form or in double-stranded helix. Herein, the nucleic acid derivative may be 5-(N-benzylcarboxamide)-2'deoxyuridine (Bz-dU) or 5-(N-(1-naphthylmethyl)carboxamide)-2'-deoxyuridine (Nap-dU), and may be known in the art or have a form modified by those skilled in the art within a range in which activity of the aptamer is not inhibited. The term "nucleic acid molecule" and, in particular, DNA or RNA molecule refers only to the primary and secondary structure of the molecule and is not limited to any specific tertiary form. Therefore, the term includes, inter alia, linear or circular DNA molecules (e.g., restriction enzyme fragments), plasmids, supercoiled DNA and double-stranded DNA found as chromosomes. When discussing the structure of a specific double-stranded DNA molecule, the sequence herein may be described in accordance with the general protocols of presenting the sequence only in the 5' to 3' direction along the un-transcribed DNA strand (i.e., the strand having a sequence matching the mRNA).

Oligonucleotides may be labeled with, for example, Biotin-nucleotide or Cy3-nucleotide to which a label such as biotin or Cy3 is covalently linked. Labeled oligonucleotides may be used as probes to detect the presence of a target. Oligonucleotides (one or all of which may be labeled) may be used as PCR primers for cloning of a full-length nucleic acid or fragments thereof, for DNA sequencing, or to detect the presence of a nucleic acid. Oligonucleotides can also be used to form a triple helix with DNA molecules. In general, oligonucleotides are prepared synthetically, and preferably, in a nucleic acid synthesizer. Therefore, oligonucleotides can be prepared using non-naturally occurring phosphoester analog linkages, such as thioester linkages.

As used herein, the terms "anti-cancer" and "cancer prevention or treatment" include any action of preventing or treating cancer, the terms "treat", "treating" and "treatment" mean any action that improves or beneficially alters symptoms of cancer by administration of the aptamer of the present invention, and the terms "prevent," "preventing," and "prevention" refer to a reduction in an occurrence of one or more diseases in an animal. The prevention described above may be complete, for example, may achieve complete absence of symptoms in a subject. The prevention may also be partial such that occurrence of symptoms in the subject is less than that would have occurred without the present invention.

As used herein, the term "polymerase chain reaction" is abbreviated as "PCR" and refers to an *in vitro* method for enzymatically amplifying a specific nucleic acid sequence. PCR may include a repeated series of temperature cycles, each cycle that consists of three steps: denaturation of a template nucleic acid to separate the strands of the target molecule; annealing to bind a single-stranded PCR oligonucleotide primer to the template nucleic acid; and extension of the bound primer by DNA polymerase. PCR provides a means for detecting the presence of a target molecule and a means for determining a relative amount of the target molecule in a nucleic acid available under quantitative or semi-quantitative conditions.

The present invention relates to a pharmaceutical composition which includes an effective amount of one or more aptamers that specifically bind to PD-L1, and optionally, in combination with a pharmaceutically acceptable carrier, excipient or additive.

The aptamer or a functional fragment thereof included in the pharmaceutical composition of the present invention may be further bound to at least one of a small molecule, a nucleic acid, a protein and a radioisotope at 5'-terminus, 3'-terminus or both thereof.

The small molecule, nucleic acid, protein and radioisotope may be directly bound to the 5'-terminus, 3'-terminus or both thereof in the aptamer or the functional fragment thereof, or may be bound through a linker.

Based on the specific binding of the aptamer or a functional fragment thereof to a target, the aptamer or the functional fragment thereof to which at least one of the small molecule, nucleic acid, protein and radioisotope is bound may exhibit cancer therapeutic effects.

For example, based on the specific binding of the aptamer or the functional fragment thereof to PD-L1, the aptamer or the functional fragment thereof to which the small molecule, nucleic acid, protein and radioisotope are bound, may exhibit therapeutic effects on cancers in which PD-L1 is over-expressed.

The small molecule, nucleic acid, protein and radioisotope that can bind to the aptamer or the functional fragment thereof is not limited as long as they can exhibit excellent cancer treatment effects by binding to the aptamer or the functional fragment thereof.

The small molecule, nucleic acid, protein and radioisotope that can bind to the aptamer or the functional fragment thereof may be known substances used for treatment of cancers in which PD-L1 is over-expressed.

As the small molecule, those commonly used in the field of anticancer drug technology may be used. For example, the small molecules may be selected from paclitaxel, monomethyl auristatin E (MMAE), monomethyl auristatin F (MMAF), cytarabine, gemcitabine, maytansine, mertansine, calicheamicin, doxorubicin, duocarmycin, pyrrolobenzodiazepine (PBD), 7-ethyl-10-hydroxy-camptothecin, tubulysin analog, 5-azacytidine, 5-fluorouracil, actino mycin D and derivatives thereof, but they are not limited thereto.

The nucleic acid may be selected from, for example, mRNA, siRNA, shRNA, miRNA and other aptamers.

The protein may be selected from, for example, therapeutic antibodies or fragments thereof such as alemtuzumab, bevacizumab, cetuximab, gemtuzumab, ibritumomab, panitumumab, rituximab, tositumomab and trastuzumab.

The radioisotopes may be selected from, for example, ³²P, ⁶⁷CU, ⁸⁹Sr, ⁹⁰Y, ^{99m}Tc, ⁹⁹MO, ¹¹¹In, ¹³¹I, ¹⁵³Sm, ¹⁶⁶Ho, ¹⁷⁷Lu, ¹⁸⁶Re, ¹⁸⁸Re, ¹⁹⁸ Au, ²¹¹At, ²¹²Bi, ²¹³Bi, ²²³Ra and ²²⁵Ac, but they are not limited thereto.

When used in combination with other active ingredients, the pharmaceutical composition according to the present invention may be formulated separately in order to be administered simultaneously or at a time interval, or may be formulated as a single composition.

The composition of the present invention may be administered once daily, twice a day, once in 2 days, once in 3 days, once in 4 days, once in 5 days, once in 6 days, once in 7 days, one every 2 weeks, once every 3 weeks, once every 4 weeks, once every 2 months, once every 6 months, or once a year, but the dosing interval is adjustable according to the needs of the patient. For longer dosing intervals, sustained release formulations may be used.

The composition of the present invention may be used to treat cancer. In one embodiment of the present invention, the composition according to the present invention may be administered for a period of more than 2 weeks, 3 weeks, 1 month, 2 months, 3 months, 4 months, 5 months, 6 months, 1 year, 2 years, 3 years, 4 years, 5 years, 10 years or 15 years. Further, in one embodiment, the inventive composition may be administered for the life of the patient. In one embodiment of the present invention, the dosage of the composition may be appropriately adjusted by monitoring the progress of the patient's disease. Those skilled in the art will be able to determine a pharmaceutically effective amount of the composition based on the condition or severity of the patient to be treated, the regimen to be applied, the pharmacokinetic characteristics of the drug to be used, and the patient who receives the composition of the present invention.

A surgical excision operation may be performed to remove the tumor, and the composition of the present invention may be administered before or after the resection. In some embodiments, the composition according to the present invention may be administered prior to a period of 1, 2, 3, 4, 5, 6, 7, 8, 9, 10, 11, 12, 13, 14 or 15 weeks or more prior to the resection; or 1, 2, 3, 4, 5, 6, 7, 8, 9, 10, 11, 12, 13, 14, 15 weeks or more after the resection.

The pharmaceutical composition of the present invention may be administered in various dosage forms. In one embodiment of the present invention, the pharmaceutical composition including an aptamer of the present invention may be formulated in a form suitable for oral, rectal, parenteral, nasal or transdermal administration, or administration by inhalation or suppository.

The administration method is not particularly limited thereto, but any oral or parenteral administration method may be used and systemic administration or local administration is also possible. However, systemic administration is more preferable and parenteral administration is most preferable. Administration into the body of a patient is preferably intravenous.

The pharmaceutical composition of the present invention may be formulated by any conventional method known to those skilled in the art. For example, if necessary, it may be used parenterally in the form of a sterile solution with water or other pharmaceutically acceptable liquid, or the injection form of a suspension. For example, in desirable combination with a pharmaceutically acceptable carrier or medium, specifically, sterile water or physiological saline, vegetable oil, emulsifier, suspending agent, surfactant, stabilizer, excipient, vehicle, preservative, binder, etc., generally, the composition may be mixed and formulated in a unit dosage form required for pharmaceutical practice generally recognized in the practical field of pharmaceutical industry. In the above formulation, an effective amount of active ingredient is so that an appropriate dose could be obtained within the indicated range.

Further, a sterile composition for injection may be prescribed according to the conventional formulation practice using an excipient such as distilled water for injection. Examples of the aqueous solution for injection may include physiological saline, isotonic solutions containing glucose and other adjuvants such as D-sorbitol, D-mannose, D-mannitol and sodium chloride. Therefore, the above aqueous injection solution may be used in combination with suitable dissolution aids, for example, alcohol, specifically, ethanol, polyalcohol such as propylene glycol, polyethylene glycol, nonionic surfactant such as polysorbate 80(TM), HCO-50, etc. Examples of an oily liquid may include sesame oil and soybean oil, and may be used in combination with benzyl benzoate and benzyl alcohol as a dissolution aid.

The injection formulation may be administered by, for example, intravenous injection, intraarterial injection, selective intraarterial injection, intramuscular injection, intraperitoneal injection, subcutaneous injection, intratumoral injection, intraventricular injection, intracerebral injection, intramedullary injection, etc., but intravenous injection is preferably used.

Further, the composition of the present invention may also be combined with buffers such as phosphate buffers or sodium acetate buffers, soothing agents such as procaine hydrochloride, stabilizers such as benzyl alcohol or phenol, and/or antioxidants. The prepared injection solution is usually filled in an appropriate ampoule.

Suspensions and emulsions may contain, as carriers, for example, natural gum, agar, sodium alginate, pectin, methylcellulose, carboxymethylcellulose or polyvinyl alcohol. Suspensions or solutions for intramuscular injection, together with the active compound, may include a pharmaceutically acceptable carrier, for example, sterile water, olive oil, ethyl oleate, glycol such as propylene glycol, and if necessary, a suitable amount of lidocaine hydrochloride.

The pharmaceutical composition according to the present invention may further include a pharmaceutically acceptable carrier. The "pharmaceutically acceptable carrier" is a diluent, adjuvant, excipient or vehicle, which is used when administering the aptamer of the present invention. Such carriers are approved by a national regulatory agency or listed in the commonly known pharmacopeias for use in animals, and more specifically, humans. The pharmaceutical carrier as described above may be in a liquid form such as water or oil, wherein the oil includes petroleum, animal, plant or synthetic-derived oils, specifically, corresponding to peanut oil, soybean oil, mineral oil, sesame oil and the like. Pharmaceutically acceptable carriers include saline, gum acacia, gelatin, starch paste, talc, keratin, colloidal silica, urea and the like. When administered to a patient, the compound of the present invention and a pharmaceutically acceptable carrier are preferably sterile. Salt solutions, liquid dextrose and glycerol solutions may also be used as the liquid carriers, specifically, as an injectable solution. Suitable pharmaceutical carriers may further contain additives such as glucose, lactose, sucrose, glycerol monostearate, sodium chloride, glycerol, propylene, glycol, water and ethanol. The composition of the present invention may contain a small amount of a wetting agent, an emulsifying agent and/or a pH buffering agent as needed. The composition of the present invention may have the form of solution, emulsion, sustained release formulation or other formulations suitable for use.

The active ingredient according to the present invention may be contained in a pharmaceutically acceptable carrier or diluent in an amount sufficient to deliver a pharmaceutically effective amount to a patient, so as not to cause side effects or serious toxicity while treating a target disease.

An oral composition generally includes an inert diluent or an edible carrier. These may be in the form of gelatin capsules or compressed tablets. For oral therapeutic administration, the active substance or its precursor may be used in the form of a tablet, oral tablet, or capsule in combination with an excipient. Pharmaceutically acceptable binders and/or adjuvants may be included as a part of the composition.

Tablets, pills, capsules, oral tablets, and the like may contain: binders such as microcrystalline cellulose, gum tragacanth or gelatin; excipients such as starch or lactose; dispersing agents such as alginic acid or corn starch; lubricants such as magnesium stearate; glydents such as colloidal silicon dioxide; sweetening agents such as sucrose or saccharin; or flavoring agents such as peppermint, methyl salicylate or orange flavorings. When the unit dosage form is a capsule, it may contain a liquid carrier such as fatty acid oil, in addition to the materials discussed herein. Further, the unit dosage form may contain various other substances such as sugar or shellac in order to alter physical properties of the dosage form.

Oral dosage forms suitable for the present invention may include capsules or tablets containing the desired active ingredient; powder or granules; suspensions in aqueous or non-aqueous liquid phases; an oil-in-water liquid emulsion or a water-in-oil emulsion; or pills.

Tablets may be prepared via compression or molding, optionally with one or more adjuvants. Compressed tablets may be prepared by compressing the active ingredient in the form of power or granules in a suitable machine, and optionally, may be admixed with binders, lubricants, inert diluents, preservatives, surfactants or dispersants. Molded tablets may be formed by molding a mixture of powdered material moistened with an inert liquid diluent in a suitable device. Further, tablets may be optionally coated and formulated to allow slow delivery of the active ingredient.

The active ingredient of the present invention may be administered in the form of elixirs, dispersions, syrups, wafers, and chewing gums. The syrup may further contain a sweetening agent such as sucrose or fructose, preservatives, dyes, and seasonings, in addition to the active ingredient.

For ophthalmic, parenteral, endothelial, subcutaneous, or topical application, the solution or suspension may be formulated for infusion while including: sterile water such as water, saline, fixed oils, polyethylene glycol, glycerin, propylene glycol or other synthetic solvents; antibacterial agents such as benzyl alcohol or methyl paraben; antioxidants such as ascorbic acid or sodium bisulfite; chelating agents such as ethylenediamine tetraacetic acid; buffers such as acetate, citrate or phosphate; and tonic agents such as salt or dextrose.

In one embodiment, the composition may be prepared with a carrier to prevent rapid elimination of the active ingredient from the body, such as a sustained release formulation including an implant and a microencapsulated delivery system. Biodegradable and biocompatible polymers such as ethylene vinyl acetate, polyanhydrides, polyglycolic acid, collagen, atelocollagen, polyorthoesters, polylactic acid, and poly(lactic-co-glycolic acid) (PLGA) may be used. Methods for manufacturing such formulations are well known to those skilled in the art.

Persons of ordinary skill in the art, that is, those skilled in the art will understand that formulations other than tablets may also be used to release the active ingredient slowly or at a controlled rate. Such formulations may be, for example, capsules, granules, and gel-caps, but it is not limited thereto.

A liposomal suspension is also a pharmaceutically acceptable carrier. This may be prepared according to any conventional method known in the art.

Cancers possibly treated using the aptamer according to the present invention may include prostate cancer, gallbladder cancer, intrahepatic biliary tract cancer, biliary tract cancer, oral cancer, pharyngeal cancer, laryngeal cancer, tongue cancer, duodenal cancer, eye tumor, mediastinal cancer, sinus cancer, renal pelvic cancer, heart cancer, glioblastoma, neuroblastoma, liver cancer, bone cancer, pancreatic cancer, skin cancer, head and neck cancer, breast cancer, lung cancer, skin or intraocular malignant melanoma, kidney cancer, uterine cancer, ovarian cancer, colon cancer, rectal cancer, anal region cancer, colorectal cancer, stomach cancer, testicular cancer, fallopian tube carcinoma, endometrial carcinoma, cervical carcinoma, vaginal carcinoma, vulvar cancer, non-Hodgkin's lymphoma, esophageal cancer, small intestinal cancer, endocrine system cancer, thyroid cancer, parathyroid cancer, adrenal gland cancer, soft tissue sarcoma, urethral cancer, penile cancer, childhood cancer, lymphocytic lymphoma, bladder cancer, ureter cancer, renal pelvic carcinoma, central nervous system (CNS) cancer, primary CNS lymphoma, tumor angiogenesis, spinal cancer, brainstem glioma, pituitary adenoma, Kaposi's sarcoma, epidermal cancer, squamous cell carcinoma, multiple myeloma, B-cell lymphoma, Hodgkin's lymphoma, acute myeloid lymphoma, chronic myeloid leukemia, chronic lymphocytic leukemia, follicular lymphoma, diffuse large B-cell lymphoma, Burkitt's lymphoma, immune large-cell lymphoma, progenitor B-lymphoblastic lymphoma, mantle cell lymphoma, acute lymphoblastic lymphoma, mycosis fungoides, anaplastic large-cell lymphoma, acute myeloid leukemia, acute lymphoblastic leukemia, hepatoblastoma, retinoblastoma, peritoneal cancer, brain tumor, thymic cancer, T-cell lymphoma or precursor T-lymphoblastic lymphoma, and any combination of the above cancers. The present invention is also applicable to treatment of metastatic cancers.

The active ingredient according to the present invention may be contained in a pharmaceutically acceptable carrier or diluent in an amount sufficient to deliver a pharmaceutically effective amount to a patient, so as not to cause side effects or serious toxicity while treating a target disease.

An embodiment of the present invention provides a composition for detecting cancer cells in which PD-L1 is over-expressed. The composition may include one or more aptamers or functional fragments thereof, the aptamers including at least one nucleic acid sequence selected from the group consisting of SEQ ID NOs: 1, 2, 10 and 18.

The aptamer or the functional fragment thereof may further include a detection label linked to, for example, 5'-terminus, 3'-terminus or both thereof, so as to detect a cancer cell in which PD-L1 is over-expressed. The detection label used herein may include, for example, a fluorescent label, a Raman scattering label, an enzyme label, an infrared label or a radiolabel.

That is, at least one selected from detection labels, such as a fluorescent label, a Raman scattering label, an enzyme label, an infrared label and a radiolabel may be additionally bound to the 5'-terminus, 3'-terminus or both thereof in the aptamer or the functional fragment thereof.

The detection label may be directly bound to the 5'-terminus, 3'-terminus or both thereof in the aptamer or the functional fragment thereof, or may be bound through a linker.

Based on the specific binding of the aptamer or the functional fragment thereof to a target, the aptamer or the functional fragment thereof to which a detection label is bound may exhibit excellent target detection effects.

For example, the aptamer or the functional fragment thereof to which a detection label is bound, based on specific binding of the aptamer or the functional fragment thereof to PD-L1, may effectively detect cancer cells in which PD-L1 is over-expressed.

Among the fluorescent labels, one commonly used in the art may be used. Fluorescent labels may include, without particular limitation thereof, fluorophores which can be introduced by commercially available oligonucleotide solid-phase synthesis services, for example, green fluorescein protein (GFP), fluorescent protein, fluorescein isothiocyanate (FITC), 6-carboxyfluorocein-aminohexyl, fluorescein derivatives, Alexa Fluor 350, Alexa Fluor 405, Alexa Fluor 488, Alexa Fluor 647, Alexa Fluor 750, Rhodamine, 6-Carboxytetramethylrhodamine, Phycoerythrin (PE), Phycocyanin (PC), PC5, PC7, Cy pigment, Cy3, Cy3.5, Cy5, Cy5.5, Cy7, TexasRed, allophycocyanin (APC), aminomethylcoumarin acetate (AMCA), Marina Blue, Cascade Blue, Cascade Yellow, Pacific Blue, SPRD, tetramethylrhodamine isothiocyanate (TRITC), R110, mC1B, CellTracker pigment, CFSE, JC-1, PKH, DCFH-DA, DHR, FDA, Calcein AM, nitrobenzoxadiazole (NBD) group, dimethylamino sulfonylbenzoxadiazole group, acridine (Acd), dansyl (Dns), 7-dimethylaminocoumarin-4-acetic acid (DMACA), 5-((2-aminoethyl)amino)naphthalene-1-sulfonic acid (EDANS), naphthalene, anthracene, protoporphyrin 9 or the like.

Further, a quencher (quenching material) absorbing fluorescent energy emitted by a fluorescent material may be additionally combined at the vicinity of the fluorescent material. In this embodiment, fluorescence may be detected by separating the fluorescent material from the quencher during the detection reaction.

Examples of the enzyme label used herein may be β-galactosidase, β-glucosidase, alkaline phosphatase, peroxidase, and malate dehydrogenase. Moreover, as a luminescent substrate, luminol, luminol derivatives, luciferin, lucigenin, etc. may be used as a labeling agent.

The Raman scattering label may use a substituent having a binding ability with a metal surface at the 5'-terminus, 3'-terminus or both thereof in the fluorescence-labeled aptamer. For example, cell recognition is performed by introducing, without particular limitation thereof, a thiol (SH) group, an alkylamino group, an aromatic amino group or a carboxyl group and adsorbing the same to the surface of, for example, gold nanoparticles so as to detect Raman scattered light emitted from aptamer-adsorbed metal particles. In this case, although it is not specifically limited, gold, silver, copper, iron, silicon, quantum dots, etc. may be used as the metal nanoparticles.

The radiolabel may use a radioisotope capable of binding to a 5'-terminus or 3'-terminus, or in the form of complementary sequence of the aptamer. For example, ¹²³I, ¹²⁴I, ¹²⁵I, ¹³¹I, ⁶⁴Cu, ⁶⁷Cu, ⁸⁹Zr, ⁹⁰Y, ¹⁵³Sm, ¹⁷⁷Lu, ¹⁸⁶Re, ¹⁸⁸Re, ¹¹¹In ^{99m}Tc, ³²P, ³⁵S, ¹⁸F, ⁶⁷Ga or ²⁰¹Tl may be introduced, but it is not limited thereto. A radiolabel aptamer based on a specific binding to a target exhibits excellent sensitivity, and may be used as a radiopharmaceutical product for molecular imaging devices.

### EXAMPLE

### Example 1. Preparation of aptamers

### 1.1 Selection of PD-L1-specific aptamer

Using systematic evolution of ligands by exponential enrichment (SELEX) method, an aptamer specific to PD-L1 protein was selected from a nucleic acid library having 40 consecutive random nucleotide sequences in the central portion and 5' primer and 3' primer binding sites at both ends, respectively:

### 1.1.1 Construction of library containing modified nucleic acids for SELEX

First, a single-stranded nucleic acid library template was synthesized by linking biotin in front of the 5' primer binding site using an automatic DNA synthesizer [Biotin-GGCTGGTGGTGTGGCTG-N(40)-CAGGCAGACGGTCACTC (SEQ ID NO: 20)] and, after PAGE purification, was used in an experiment (Trilink, USA).

In order to construct a nucleic acid library including modified nucleic acid 5-(N-benzylcarboxamide)-2' deoxyuridine (Bz-dU) or 5-(N-(1-naphthylmethyl)carboxamide)-2'-deoxyuridine (Nap-dU), the library template was immobilized on the resin that was surface-modified with a streptavidin protein binding to biotin of the library template. Specifically, the library template and 500 µL resin (Thermo Scientific, USA) were mixed in 5 M NaCl solution and reacted at room temperature for 1 hour to perform immobilization. To the resin washed with NaCl, 20 µM 5' primer (GAGTGACCGTCTGCCTG (SEQ ID NO: 21)), 0.5 mM dNTP (ATP, GTP, CTP, Nap-dUTP or Bz-dUTP), 0.25 U/µL KOD XL DNA polymerase, 120 mM Tris-HCl pH7.8, IX KOD buffer and 0.1mg/ml BSA were added, followed by enzymatic reaction at 70°C for 2 hours to prepare double-stranded DNA containing modified nucleic acids. Then, the double-stranded DNA was denatured using 20 mM NaOH to separate the resin and ssDNA, neutralized with 80 mM HCl solution, concentrated using Amicon ultra-15 (Merck Millipore, USA), and quantified with a UV spectrophotometer to eventually construct a single-stranded library containing modified nucleic acids.

### 1.1.2 Selection of aptamers specific to immune anticancer target proteins

As a target protein for SELEX, a protein containing a histidine tag (6xHis-tag) (R&D, USA) among recombinant proteins was used in the experiment. The target protein was surface-modified with cobalt and immobilized on beads using Dynabead (Thermo Scientific, USA), which is a magnetic bead that binds to the His-tag of the protein. Specifically, a target protein and 20 µg of magnetic beads dissolved in the selection buffer (40 mM HEPES, 102 mM NaCl, 5 mM KCl) were reacted in a thermomixer (Thermomixer; Eppendorf, Germany) at 25 °C and 1200 rpm for 30 minutes, then washed with the selection buffer using an external magnet to prepare the product immobilized on the beads.

The synthesized modified nucleic acid-containing library was placed in the selection buffer and the temperature was gradually lowered from 95 °C to 37 °C while reacting for 10 seconds, so as to induce formation of a tertiary structure of the nucleic acid library. For negative selection, a protein competition buffer (10 µM prothrombin, 10 µM casein, 0.1% (w/v) albumin) was mixed with the above reaction solution, and then added to 20 µg of magnetic beads, followed by reacting the same using a thermomixer at 37 °C and 1200 rpm for 10 minutes. Thereafter, the supernatant was transferred to the Dynabead to which the target protein was immobilized, followed by aptamer selection. Specifically, the modified nucleic acid library after negative selection was reacted with target protein-immobilized magnetic beads in the thermomixer at 37 °C and 1200 rpm for 1 hour. After washing DNA and the target protein-magnetic bead complex with a selection buffer 5 times, 2 mM NaOH solution was added to recover the DNA pool combined with the target protein, followed by neutralization using 8 mM HCl solution.

In order to use the DNA pool combined with the target protein in the next round of SELEX, it was amplified using QPCR (quantitative PCR, CFX real time PCR detection system; Biorad, USA). The recovered DNA pool in the SELEX process was mixed with QPCR buffer [(5' primer (GAGTGACCGTCTGCCTG (SEQ ID NO: 21)), 3' primer (Biotin-GGCTGGTGGTGTGGCTG (SEQ ID NO: 22)), IX KOD buffer, KOD XL enzyme, 1 mM dNTP], and then, one cycle under conditions of 96 °C for 15 seconds, 55 °C for 10 seconds and 68 °C for 30 minutes was conducted, followed by repeating 30 cycles at 96 °C for 15 seconds and 72°C for 1 minute, so as to prepare a double-stranded oligoDNA with amplification of DNA pool combined with the target protein.

The double-stranded oligoDNA formed through QPCR was mixed with 25 µl of streptavidin Myone magnetic beads (Thermo Scientific, USA) at room temperature for 10 minutes and immobilized. Then, 20 mM NaOH solution was added to remove antisense strands, followed by synthesizing a DNA pool containing modified nucleic acid using an enzyme in the same way as used for construction of the library containing the modified nucleic acid, and then, using the same in the next round. The above SELEX round was performed for a total of 8 times.

### 1.1.3 Measurement of binding affinity (Kd) of SLEX DNA pool

In order to determine the binding force (i.e., binding affinity) between the target protein and the DNA pool subjected to the SELEX round, a filter binding assay was conducted. First, the ends of DNA pools at 6^{th} round and 8^{th} round were labeled using α-³²P ATP (Perkin almeo, USA) and TdT (Terminal deoxynucleotidyl transferase; New England Bio Labs, USA). After a reaction of 1 µM oligoDNA obtained through the SELEX process at 37 °C for 30 minutes under conditions of 0.25 µL α-³²P ATP, 0.25 µL TdT and NEB buffer 4, the reaction product was treated at 70 °C for 10 minutes to inactivate the TdT enzyme. The labeled oligoDNA pool was purified using a Micro spin G50 column (GE Healthcare, USA), and then 20,000 cpm of the DNA pool was added to 100 µL selection buffer and cooled slowly from 95 °C to 37 °C by 0.1 °C per second. After serial dilution of the target protein using a selection buffer, 30 µL of the heated and cooled labeled oligoDNA pool was added and reacted at 37 °C for 30 minutes. After spotting 2 µL of a mixture including the labeled oligoDNA and the target protein on a nylon membrane (GE Healthcare, USA), 5.5 µL of zorbax resin (Agilent, USA) was added. Then, the prepared product was put into a Durapore filter (Millipore, USA) moistened with 50 µL of selection buffer in advance and the solution was removed by vacuum, followed by washing the membrane filter with 100 µL of selection buffer. After exposing the filter plate to an image plate for 16 hours, the image was quantified with FLA-5100 (Fujifilm, Japan). Table 1 shows a binding affinity between the obtained target protein and the SELEX DNA pool. The binding affinity was estimated from the value obtained through the filter binding assay by means of Sigmaplot 11 analysis software (Systat Software, USA), and Bmax in Table 1 represents a ratio of the bound aptamer compared to the input, while Kd (dissociation constant) indicates affinity.

**[TABLE 1]**

| | Bz lib | 6R Bz | 8R Bz | Nap lib | 6R Nap | 8R Nap |
|---|---|---|---|---|---|---|
| Bmax | 0.02 | 0.00 | 0.30 | 0.09 | 0.25 | 0.14 |
| Kd | 0.71 | 3.53 | 1.61 | 0.64 | 3.08 | 0.74 |

Strong binding affinity was observed in all three samples except for 6^{th} round SELEX using a library containing the modified nucleic acid Bz-dU.

### 1.1.4 Base sequence analysis of SLEX DNA pool

After amplification of 8^{th} round sample of the modified nucleic acid among the SELEX round DNA pool with confirmed binding affinity through PCR using a 5' primer (5' primer GAGTGACCGTCTGCCTG (SEQ ID NO: 21)) and 3' primer (GGCTGGTGGTGTGGCTG (SEQ ID NO: 22)), the amplified product was cloned using TOPO TA Cloning kit (Thermo Scientific, USA) and transformed into *E. coli* TOP10 cells. After checking whether the PCR product cloned from a single colony grown on an agar medium was inserted using an RCA kit (Engnomics, Korea), nucleotide sequence analysis (Solgent, Korea) was performed using a RCA sample of the clone having a single sequence inserted therein by a BigDye terminator cycle sequencing kit (Thermo Scientific, USA) and capillary electrophoresis. Eventually, 50 DNA molecule sequences present in the concentrated DNA pool were confirmed through SELEX.

### 1.1.5 Biosynthesis of clonal aptamers and measurement of binding affinity

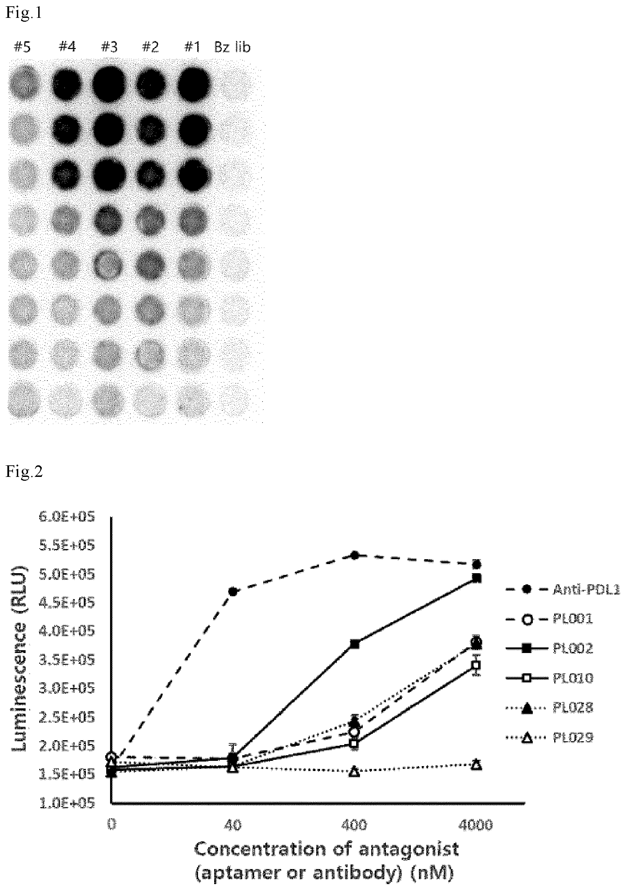

The 50 nucleotide sequences that have been analyzed were again subjected to analysis using the Multalin web site, and 5 aptamer sequences with the highest expression frequency were selected (Table 2, clone sequence). For the five selected aptamers, each aptamer was biosynthesized using the double-stranded DNA used for nucleotide sequence analysis as a template, as well as the 5' primer (GAGTGACCGTCTGCCTG (SEQ ID NO: 21)) and the biotin bound 3' primer (Biotin-GGCTGGTGGTGTGGCTG (SEQ ID NO: 22)) in the same manner as the DNA pool binding affinity measurement method described above, followed by measuring the binding affinity to the target protein was measured (FIG. 1). As a result, aptamers with excellent binding force that exhibited a dissociation constant (Kd) of 0.18 to 1.36 nM in the four sequences were selected (Table 3).

**[TABLE 2]**

| Name (SEQ ID No.) | Sequence (5'-> 3') 5:Bz-dU | Length (mer) |
|---|---|---|
| PL001 (SEQ ID NO: 1) | GAGTGACCGTCTGCCTGA5GAAAAG55555GA55G5AC C55AG5GCAC5CAACAG5GCAGCCACACCACCAGCC | 74 |
| PL002 (SEQ ID NO: 2) | GAGTGACCGTCTGCCTG5G5A5GCCGCACGA5CGCCC5 A5555GCGAG555AC5CACAGCCACACCACCAGCC | 73 |
| PL010 (SEQ ID NO: 10) | GAGTGACCGTCTGCCTG55AAGCGGC5AAG55555GA55 G5ACC55AG5GCCC55ACCAGCCACACCACCAGCC | 74 |
| PL028 (SEQ ID NO: 18) | GAGTGACCGTCTGCCTG5G5A5GCCGCACGA5CGCCC5 A5555GCGAG555AAC5GACAGCCACACCACCAGCC | 74 |
| PL029 (SEQ ID NO: 19) | GAGTGACCGTCTGCCTGCCA555CC5GC55GC5C55CC5 CC555A55555CACC555CAGCCACACCACCAGCC | 74 |

**[TABLE 3]**

| Target protein | PD-L1 | | | | | |
|---|---|---|---|---|---|---|
| Name | PL029 (#5) | PL028 (#4) | PL010 (#3) | PL002 (#2) | PL001 (#1) | Bz Lib |
| Bmax | 0.10 | 0.06 | 0.06 | 0.05 | 0.07 | 0.02 |
| Kd (nM) | 243.08 | 1.36 | 0.93 | 0.18 | 1.10 | 0.52 |

**1.1.6 Synthesis of aptamers:** An aptamer containing a modified nucleic acid, Bz-dU, was synthesized using the five clone sequences (Table 2). The aptamer was synthesized by a general automatic DNA synthesis method using MerMade 12 (Bioautomation, USA). The synthesized aptamer was used in the experiment after HPLC purification.

### 1.1.7 Measurement of PD-1/PD-L1 binding inhibition of aptamers

Inhibition of PD-1/PD-L1 binding of the synthetic aptamer was determined by conducting cell-based tests.

Inhibition of PD-1/PD-L1 binding at a cellular level was performed using the following kit. The cell-based PD-1/PD-L1 binding inhibition test (Promega, USA) is a luciferase repoter-based assay method, in which Jurkat effector cells with expression of PD-1 and luciferase genes, as well as CHO-K1 cells with expression of TCR activator and PD-L1 are mixed together, followed by treating and culturing the mixture with an inhibitor (aptamer or neutralizing antibody). In this case, PD-1/PD-L1 binding inhibitory activity at the cellular phase was tested. This system is a cell-based inhibitory activity measurement method that is designed to increase a fluorescent signal with increase in the inhibitory activity, and a commercially available PD-L1 neutralizing antibody was used as a positive control.

First, the inhibitory activity of the five aptamers synthesized using the clone sequence is as shown in FIG. 2. As a result of measuring cell-based inhibitory activity, PL-1/PD-L1 binding inhibition was confirmed in four aptamers except for PL029 among the five synthesized aptamers.

### 1.2 PD-L1-specific aptamer functional fragment

### 1.2.1 Preparation of PD-L1-specific aptamer functional fragment

A functional fragment was prepared using the sequence of PL001 (74mer), which is an aptamer confirmed to exhibit PD-1/PD-L1 binding inhibitory activity in Example 1.1. By reducing the length from both ends of the PL001 aptamer, an aptamer showing similar or better inhibitory activity to the clone sequence at the minimum length was selected. 50mer, 46mer, 45mer, 40mer and 24mer length aptamers were generated by cleaving the 5'-terminus and 3'-terminus of PL001, respectively (PL016, PL017, PL018, PL019, PL020 and PL009; Table 4).

**[TABLE 4]**

| Name (SEQ ID No.) | Sequence (5'-> 3') 5:Bz-dU | Length (mer) |
|---|---|---|
| PL016 (SEQ ID NO: 11) | GCCTGA5GAAAAG55555GA55G5ACC55AG5GCAC5 CAACAG5GCAGCC | 50 |
| PL017 (SEQ ID NO: 12) | GCCTGA5GAAAAG55555GA55G5ACC55AG5GCAC5 CAACAG5GC | 46 |
| PL018 (SEQ ID NO: 13) | A5GAAAAG55555GA55G5ACC55AG5GCAC5CAACA G5GCAGCC | 45 |
| PL019 (SEQ ID NO: 14) | A5GAAAAG55555GA55G5ACC55AG5GCAC5CAACA G5G | 40 |
| PL020 (SEQ ID NO: 15) | 55GA55G5ACC55AG5GCAC5CAA | 24 |
| PL009 (SEQ ID NO: 9) | AAG55555GA55G5ACC55AG5GC | 24 |

### 1.2.2 Measurement of PD-1/PD-L1 binding inhibition of aptamers

PD-1/PD-L1 binding inhibition of the synthetic fragment aptamer was determined by conducting a purified protein-based test. Purified protein-based PD-1/PD-L1 binding inhibition test (BPS, USA) was conducted by ELISA method based on horseradish peroxidase (HRP). First, purified PD-L1 protein according to the product manual was fixed on a 96 well plate, and then each well was washed with Immuno Buffer 1 for a total of 3 times in order to remove unbound PD-L1 protein. Aptamers were prepared in a selection buffer for each experimental concentration and slowly cooled from 95 °C to 37°C by 0.1°C per second. 5 µL of the aptamer was added to the well to which PD-L1 protein was fixed, followed by adding 20 µL of Biotin-bound PD-1 protein prepared according to the corresponding instructions to each well again. After washing each well with Immuno Buffer 1 for 3 times, 100 µL of Streptavidin-HRP diluted in a blocking buffer according to the corresponding instructions was added, followed by conducting a reaction for 1 hour. After adding 100 µL of the HRP substrate mixture to the well, from which the supernatant was removed, a fluorescent intensity was measured using a luminometer so as to determine the inhibition of PD-1/PD-L1 binding. IC₅₀ (50% inhibitory concentration) was calculated using the online tool (https://www.aatbio.com/tools/ic50-calculator).

**[TABLE 5]**

| Name (SEQ ID No) | PL016 (SEQ ID NO: 11) | PL017 (SEQ ID NO: 12) | PL018 (SEQ ID NO: 13) | PL019 (SEQ ID NO: 14) | PL020 (SEQ ID NO: 15) | PL009 (SEQ ID NO: 9) | PL001 (SEQ ID NO: 1) |
|---|---|---|---|---|---|---|---|
| IC₅₀ (nM) | 3.3 | 4.0 | >120 | 15.3 | >120 | 4.6 | 6.0 |

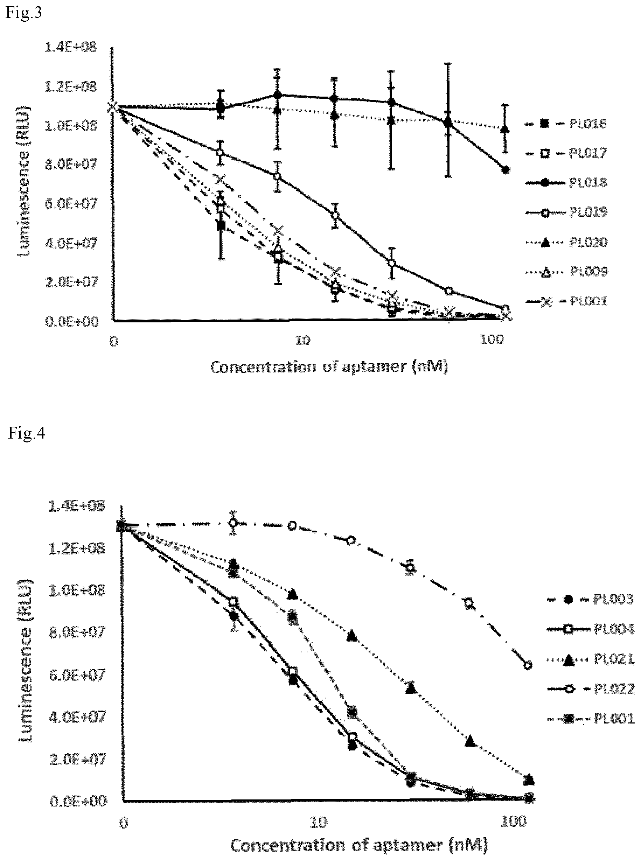

As a result of examining the binding affinity of each of the synthesized parent aptamer PL001 and the six fragment aptamers, it was confirmed that the clone sequence PL001 exhibited excellent inhibitory activity, as shown in Table 5 and FIG. 3. Further, fragments of this clone sequence, PL016, PL017 and PL009 showed inhibitory activity similar to or improved from that of the parent aptamer.

### 1.3 PD-L1-specific aptamer functional fragment

### 1.3.1 Preparation of PD-L1-specific aptamer functional fragment

In order to screen an aptamer having better PD-1/PD-L1 binding inhibitory activity in a fragment with a smaller length, a functional fragment was further prepared using a sequence of PL001 (74mer) which is an aptamer confirmed to exhibit PD-1/PD-L1 binding inhibitory activity in Example 1.1. By cleaving the 5'-terminus and 3'-terminus of PL001, respectively, 34mer, 29mer, 23mer, and 22mer aptamers were generated (PL003, PL004, PL021, and PL022, respectively; Table 6).

**[TABLE 6]**

| Name (SEQ ID No.) | Sequence (5'-> 3') 5:Bz-dU | Length (mer) |
|---|---|---|
| PL003 (SEQ ID NO: 3) | GCCTGA5GAAAAG55555GA55G5ACC55AG5GC | 34 |
| PL004 | A5GAAAAG55555GA55G5ACC55AG5GC | 29 |
| (SEQ ID NO: 4) | | |
| PL021 (SEQ ID NO: 16) | AAG55555GA55G5ACC55AG5G | 23 |
| PL022 (SEQ ID NO: 17) | AG55555GA55G5ACC55AG5G | 22 |

### 1.3.2 Measurement of PD-1/PD-L1 binding inhibition of aptamers:

Aptamer PD-1/PD-L1 binding inhibitory activity described in Table 6 of Example 1.3.1 was measured in the same manner as in Example 1.2.2.

**[TABLE 7]**

| Name (SEQ ID No.) | PL003 (SEQ ID NO: 3) | PL004 (SEQ ID NO: 4) | PL021 (SEQ ID NO: 16) | PL022 (SEQ ID NO: 17) | PL001 (SEQ ID NO: 1) |
|---|---|---|---|---|---|
| IC₅₀ (nM) | 6.3 | 7.0 | 27.7 | >120 | 10.5 |

Consequently, as shown in Table 7 and FIG. 4, it was confirmed that PL003 and PL004 exhibit superior inhibitory activity than the parent aptamer PL001.

### 1.4 PD-L1-specific aptamer functional fragment

### 1.4.1 Preparation of PD-L1-specific aptamer functional fragment

In order to screen various aptamers with superior PD-1/PD-L1 binding inhibitory activity, aptamers PL005, PL006, PL007 and PL008 were prepared by cleaving the 5'-terminus and 3'-terminus of PL002 of Example 1.1, respectively (Table 8).

**[TABLE 8]**

| Name (SEQ ID No.) | Sequence (5'-> 3') 5:Bz-dU | Length (mer) |
|---|---|---|
| PL002 (SEQ ID NO: 2) | GAGTGACCGTCTGCCTG5G5A5GCCGCACGA5CGCC C5A5555GCGAG555AC5CACAGCCACACCACCAGCC | 73 |
| PL005 (SEQ ID NO: 5) | GCCTG5G5A5GCCGCACGA5CGCCC5A5555GCGAG55 5AC5CACAGCC | 49 |
| PL006 (SEQ ID NO: 6) | 5G5A5GCCGCACGA5CGCCC5A5555GCGAG555AC5C A | 39 |
| PL007 (SEQ ID NO: 7) | A5GCCGCACGA5CGCCC5A5555GCGAG555AC | 33 |
| PL008 (SEQ ID NO: 8) | CCGCACGA5CGCCC5A5555GCGAG55 | 27 |

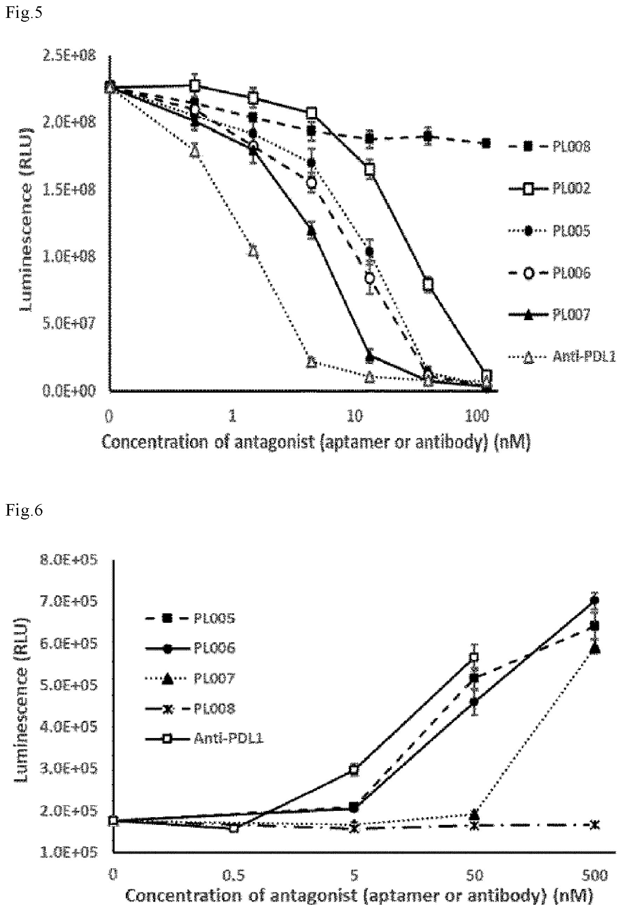

**4.2 Measurement of PD-1/PD-L1 binding inhibition of aptamer:** In the same manner as described in Example 1.2.2, the PD-1/PD-L1 binding inhibitory activity of the aptamers described in Table 8 of Example 1.4.1 was measured (FIG. 5). A commercially available PD-L1 neutralizing antibody was used as a positive control for this test.

Consequently, as shown in Table 9 and FIG. 5, it was confirmed that PL007 exhibited superior inhibitory activity than the parent aptamer PL002, while PL006 and PL005 had the next highest PD-1/PD-L1 binding inhibitory activity.

**[TABLE 9]**

| Name (SEQ ID No.) | PL002 (SEQ ID NO: 2) | PL005 (SEQ ID NO: 5) | PL006 (SEQ ID NO: 6) | PL007 (SEQ ID NO: 7) | PL008 (SEQ ID NO: 8) | Anti-PDLl |
|---|---|---|---|---|---|---|
| IC₅₀ (nM) | 31.3 | 12.6 | 9.6 | 4.7 | >120 | 1.2 |

Inhibitory activity against the four aptamer functional fragments (PL005, PL006, PL007 and PL008) described in Table 8 of Example 1.4.1 was tested using a cell-based system in the same manner as in Example 1.1.7, thereby obtaining the result of FIG. 6. The inhibitory activity was confirmed in the relatively longer PL005 and PL006. Specifically, in the case of PL005, the 50 nM treatment group showed similar inhibitory activity that was not statistically different from the PD-L1 antibody used as a positive control. The shorter PL007 had the lowest IC ₅₀ value in the purified protein system, however, showed relatively weak inhibitory activity in the cell-based system.

Table 10 below shows the aptamer sequences described in the present invention.

**[TABLE 10]**

| Name (SEQ ID No.) | Sequence (5'-> 3') 5:Bz-dU | Length (mer) |
|---|---|---|
| PL001 (SEQ ID NO: 1) | GAGTGACCGTCTGCCTGA5GAAAAG55555GA55G5A CC55AG5GCAC5CAACAG5GCAGCCACACCACCAGC C | 74 |
| PL002 (SEQ ID NO: 2) | GAGTGACCGTCTGCCTG5G5A5GCCGCACGA5CGCC C5A5555GCGAG555AC5CACAGCCACACCACCAGCC | 73 |
| PL003 (SEQ ID NO: 3) | GCCTGA5GAAAAG55555GA55G5ACC55AG5GC | 34 |
| PL004 (SEQ ID NO: 4) | A5GAAAAG55555GA55G5ACC55AG5GC | 29 |
| PL005 (SEQ ID NO: 5) | GCCTG5G5A5GCCGCACGA5CGCCC5A5555GCGAG55 5AC5CACAGCC | 49 |
| PL006 | 5G5A5GCCGCACGA5CGCCC5A5555GCGAG555AC5C | 39 |
| (SEQ ID NO: 6) | A | |
| PL007 (SEQ ID NO: 7) | A5GCCGCACGA5CGCCC5A5555GCGAG555AC | 33 |
| PL008 (SEQ ID NO: 8) | CCGCACGA5CGCCC5A5555GCGAG55 | 27 |
| PL009 (SEQ ID NO: 9) | AAG55555GA55G5ACC55AG5GC | 24 |
| PL010 (SEQ ID NO: 10) | GAGTGACCGTCTGCCTG55AAGCGGC5AAG55555GA 55G5ACC55AG5GCCC55ACCAGCCACACCACCAGCC | 74 |
| PL016 (SEQ ID NO: 11) | GCCTGA5GAAAAG55555GA55G5ACC55AG5GCAC5C AACAG5GCAGCC | 50 |
| PL017 (SEQ ID NO: 12) | GCCTGA5GAAAAG55555GA55G5ACC55AG5GCAC5C AACAG5GC | 46 |
| PL018 (SEQ ID NO: 13) | A5GAAAAG55555GA55G5ACC55AG5GCAC5CAACAG 5GCAGCC | 45 |
| PL019 (SEQ ID NO: 14) | A5GAAAAG55555GA55G5ACC55AG5GCAC5CAACAG 5G | 40 |
| PL020 (SEQ ID NO: 15) | 55GA55G5ACC55AG5GCAC5CAA | 24 |
| PL021 (SEQ ID NO: 16) | AAG55555GA55G5ACC55AG5G | 23 |
| PL022 (SEQ ID NO: 17) | AG55555GA55G5ACC55AG5G | 22 |
| PL028 (SEQ ID NO: 18) | GAGTGACCGTCTGCCTG5G5A5GCCGCACGA5CGCC C5A5555GCGAG555AAC5GACAGCCACACCACCAGC C | 74 |
| PL029 (SEQ ID NO: 19) | GAGTGACCGTCTGCCTGCCA555CC5GC55GC5C55CC 5CC555A55555CACC555CAGCCACACCACCAGCC | 74 |

For example, for the purpose of claim construction, the claims set forth below are not to be construed narrower in any way than their literal language, and therefore, exemplary embodiments based on the specification should not be read as claims. Accordingly, it is to be understood that the present invention has been described by way of illustration but is not a limitation to the scope of the claims. Therefore, the present invention is limited only by the following claims. All publications, issued patents, patent applications, books and journal articles cited in this application are each

## Claims

1. An aptamer or a functional fragment thereof, capable of specifically binding to programmed death-ligand 1 (PD-L1) or capable of inhibiting interaction between programmed cell death protein 1 (PD-1) and PD-L1 by specifically binding to PD-L1, the aptamer comprising a nucleic acid sequence of SEQ ID NO: 1, 2, 10 or 18.

2. The aptamer or a functional fragment thereof according to claim 1, wherein the functional fragment is any one of SEQ ID NOs: 3 to 9, and 11 to 17.

3. A pharmaceutical composition for treating cancer, comprising one or more of an aptamer or a functional fragment thereof, the aptamer comprising a nucleic acid sequence selected from the group consisting of SEQ ID NOs: 1, 2, 10, and 18.

4. The pharmaceutical composition according to claim 3, wherein the functional fragment is any one of SEQ ID NOs: 3 to 9, and 11 to 17.

5. The pharmaceutical composition according to claim 3, wherein the functional fragment is SEQ ID NO: 3, 4, 5, 6, 7, 9, 11, 12, 14 or 16.

6. The pharmaceutical composition according to claim 3, wherein the cancer is one in which PD-L1 is over-expressed.

7. The pharmaceutical composition according to claim 3, wherein the cancer is prostate cancer, gallbladder cancer, intrahepatic biliary tract cancer, biliary tract cancer, oral cancer, pharyngeal cancer, laryngeal cancer, tongue cancer, duodenal cancer, eye tumor, mediastinal cancer, sinus cancer, renal pelvic cancer, heart cancer, glioblastoma, neuroblastoma, liver cancer, bone cancer, pancreatic cancer, skin cancer, head and neck cancer, breast cancer, lung cancer, skin or intraocular malignant melanoma, kidney cancer, uterine cancer, ovarian cancer, colon cancer, rectal cancer, anal region cancer, colorectal cancer, stomach cancer, testicular cancer, fallopian tube carcinoma, endometrial carcinoma, cervical carcinoma, vaginal carcinoma, vulvar cancer, non-Hodgkin's lymphoma, esophageal cancer, small intestinal cancer, endocrine system cancer, thyroid cancer, parathyroid cancer, adrenal gland cancer, soft tissue sarcoma, urethral cancer, penile cancer, childhood cancer, lymphocytic lymphoma, bladder cancer, ureter cancer, renal pelvic carcinoma, central nervous system (CNS) cancer, primary CNS lymphoma, tumor angiogenesis, spinal cancer, brainstem glioma, pituitary adenoma, Kaposi's sarcoma, epidermal cancer, squamous cell carcinoma, multiple myeloma, B-cell lymphoma, Hodgkin's lymphoma, acute myeloid lymphoma, chronic myeloid leukemia, chronic lymphocytic leukemia, follicular lymphoma, diffuse large B-cell lymphoma, Burkitt's lymphoma, immune large-cell lymphoma, progenitor B-lymphoblastic lymphoma, mantle cell lymphoma, acute lymphoblastic lymphoma, mycosis fungoides, anaplastic large-cell lymphoma, acute myeloid leukemia, acute lymphoblastic leukemia, hepatoblastoma, retinoblastoma, peritoneal cancer, brain tumor, thymic cancer, T-cell lymphoma or precursor T-lymphoblastic lymphoma.

8. The pharmaceutical composition according to claim 3, wherein at least one of a small molecule, a nucleic acid, a protein and a radioisotope is bound to the 5'-terminus, 3'-terminus or both thereof in the aptamer or the functional fragment thereof.

9. A composition for detecting cancer cells, comprising at least one of an aptamer or a functional fragment thereof, the aptamer comprising a nucleic acid sequence selected from the group consisting of SEQ ID NOs: 1, 2, 10 and 18.

10. The composition for detection according to claim 9, wherein the functional fragment is any one of SEQ ID NOs: 3 to 9, and 11 to 17.

11. The composition according to claim 9, wherein the functional fragment is SEQ ID NO: 3, 4, 5, 6, 7, 9, 11, 12, 14 or 16.

12. The composition according to claim 9, wherein the cancer cells are cells in which PD-L1 is over-expressed.

13. The composition according to claim 9, wherein the cancer is prostate cancer, gallbladder cancer, intrahepatic biliary tract cancer, biliary tract cancer, oral cancer, pharyngeal cancer, laryngeal cancer, tongue cancer, duodenal cancer, eye tumor, mediastinal cancer, sinus cancer, renal pelvic cancer, heart cancer, glioblastoma, neuroblastoma, liver cancer, bone cancer, pancreatic cancer, skin cancer, head and neck cancer, breast cancer, lung cancer, skin or intraocular malignant melanoma, kidney cancer, uterine cancer, ovarian cancer, colon cancer, rectal cancer, anal region cancer, colorectal cancer, stomach cancer, testicular cancer, fallopian tube carcinoma, endometrial carcinoma, cervical carcinoma, vaginal carcinoma, vulvar cancer, non-Hodgkin's lymphoma, esophageal cancer, small intestinal cancer, endocrine system cancer, thyroid cancer, parathyroid cancer, adrenal gland cancer, soft tissue sarcoma, urethral cancer, penile cancer, childhood cancer, lymphocytic lymphoma, bladder cancer, ureter cancer, renal pelvic carcinoma, central nervous system (CNS) cancer, primary CNS lymphoma, tumor angiogenesis, spinal cancer, brainstem glioma, pituitary adenoma, Kaposi's sarcoma, epidermal cancer, squamous cell carcinoma, multiple myeloma, B-cell lymphoma, Hodgkin's lymphoma, acute myeloid lymphoma, chronic myeloid leukemia, chronic lymphocytic leukemia, follicular lymphoma, diffuse large B-cell lymphoma, Burkitt's lymphoma, immune large-cell lymphoma, progenitor B-lymphoblastic lymphoma, mantle cell lymphoma, acute lymphoblastic lymphoma, mycosis fungoides, anaplastic large-cell lymphoma, acute myeloid leukemia, acute lymphoblastic leukemia, hepatoblastoma, retinoblastoma, peritoneal cancer, brain tumor, thymic cancer, T-cell lymphoma or precursor T-lymphoblastic lymphoma.

14. The composition according to claim 9, wherein at least one of a fluorescent label, a Raman scattering label, an enzyme label, an infrared label and a radiolabel is bound to the 5'-terminus, 3'-terminus or both thereof in the aptamer or the functional fragment thereof.
